# EUROPEAN PATENT SPECIFICATION

(11) **EP 2 945 589 B1**
(45) Date of publication and mention of the grant of the patent: **17.11.2021**
(21) Application number: 14707215.1
(22) Date of filing: 16.01.2014
(51) Int. Cl.: A63B 71/00, A63B 23/035, A63B 24/00, A61B 5/389, A63B 21/005, A63B 21/00, A63B 71/02

(54) **REHABILITATION DEVICE**
REHABILITATIONSVORRICHTUNG
DISPOSITIF DE RÉÉDUCATION

(30) Priority: 17.01.2013 PL 40246513 P
(43) Date of publication of application: 25.11.2015
(73) Proprietor: Egzotech Spólka z o.o., 44-100 Gliwice (PL)
(72) Inventor: MIKULSKI, Michal, PL-44-100 Gliwice (PL)
(74) Representative: Korga, Leokadia
(86) International application number: PCT/IB2014/058339
(87) International publication number: WO 2014/111882

(56) References cited:
- WO-A1-95/26701
- WO-A2-02/096274
- US-A- 2 777 439
- None

## Description

The invention pertains to a device for rehabilitating patients especially with peripheral nervous system paralysis.

American patent application US 2006/0253052 describes a method of rehabilitating a patient suffering from total or partial paralysis of the peripheral nervous system, manifesting itself by weakened muscular tension or muscular atrophy, which entails the use of feedback in which the patient views and/or hears a signal related to the intensity of electromyographic (EMG) activity the patient produces in the appropriate muscles while attempting to move them. The method is intended mostly for highly disabled patients as an alternative for therapy using the AMES method.

American patent application US 2004/0106881 describes a powered device, which worn about a patient's elbow or other joint, senses relatively low level signals in the vicinity of the joint generated by a patient having spinal cord or other nerve damage. In response to the relatively low level signals, the powered device moves thus helping the patient to move the joint.

American patent US 5722420 describes a method of rehabilitation using closed loop traction force control based on EMG signals from selected muscle. The method is based upon a main traction controller, which uses a high signal-to-noise ratio EMG, which is then scanned and processed into units compatible with computer systems, while adaptively adjusting traction force based on real time detected EMG signals, using biological feedback control by means of visual or audio alarms. The kernel of this solution is its modality which adjusts traction force to the EMG signals detected in real time.

American patent application US 2009/0259338 describes a robotic system for rehabilitation using EMG signals, comprising EMG electrodes sequentially connected in series, a DAS card and an additional element connecting to the motor. The electrodes emit an EMG signal making the patient's muscles of the suffering joint contract, which are then measured as input and sent to the control portion of the DAS card. The control portion uses the EMG signals to calculate an appropriate moment to apply an impulse to the suffering joint to make it move. Patent application WO 02/096274 describes a portable intelligent stretching device for use by patients suffering from spastic and contractured joints and limbs. The intelligent stretching device has a motor and a motor shaft for rotating the joint or limb. The variable velocity and stretch distance of the device is determined by a torque sensor on the joint or limb that communicates information to a controller which subsequently instructs the motor as to the variable velocity and stretch distance. This stretching device uses signal from EMG to reduce speed of the motor during stretching of the joint.

American patent US 2777439 describes the manipulator being particularly useful in treating patients inflicted with the type of disease wherein appropriate exercising of various muscle or joints of the body will improve the condition and in some cases completely rehabilitate the affected people.

Patent application WO95/26701 describes an exercise, rehabilitation and extremity testing method and apparatus having a cycloidal speed reducer. A multi-position chair is rotatably mounted to a stand which is slidingly mounted to a track on a platform. A dynamometer is mounted on a pedestal on the platform such that it is capable of rotating about a horizontal axis (b) and a vertical axis (a). The vertical height of the dynamometer can also be adjusted. An input apparatus capable of engaging the limb of a user is mounted to an input shaft of the dynamometer. A motor controls the movement of the input shaft through a cycloidal speed reducer. Consequently, the motor controls the movement of the input arm and the user's limb. The user exercises his muscles by applying force to the input apparatus according to a number of different protocols.

The main aim of the hereby invention is the construction of a device enabling simultaneous rehabilitation of a few patients with peripheral nervous system paralysis by a single physiotherapist.

The invention relates to a rehabilitation device used to rehabilitate weakened muscular tension comprising a remote control terminal, a base, an extension mounted to a column with adjustable height containing an motor configured to propel the extension and at least two electrodes of the electromyography (EMG) sensor, wherein that the motor is configured to enable the extension to move within the angular range of 360°, while the EMG signal from the sensor connected to the muscle or muscles of the patient is transmitted to the network of the motor controlling module, and then further by radio or cable connection to a remote control terminal;
wherein the controlling module is configured to provide individual adjustment of the speed and rotation angle of the extension depending exclusively on the character of the signal received from the EMG and a program set in the remote control terminal.

It is advisable to mount the base on at least three wheels.

In one embodiment the middle part of the base is telescopically folded.

In another embodiment the arm of the device is telescopically folded.

Favourably, the arm is mounted detachably on the column.

Favourably, a force sensor is built in between the end of the motor and the arm mounting.

In an additional embodiment, an intermediate module is configured to display a picture on a screen depending on the movement made by the patient being rehabilitated.

It is advisable to have the exchangeable arm made in a size adjusted for rehabilitation of a specific limb.

Favourably the detachable extension has an electronic module supplied from the device that are configured to enable device to identify arm. Favourably the detachable extension of the device consists of electronic identification modules, drives (motors) and sensors, and has a mean to power it's drive and the data derived from the drive and sensors are passed to the engine module and network.

Favourably the arm can be blocked by an electromagnet device.

Favourably mechanically or electrically retractable pins, configured to lock the range of movement of the arm are arranged on the circumference of the front head of the device.

Favourably the base comprises replaceable batteries to power the device. Favourably the batteries in the base have integrated antennas for wireless charging.

Favourably the device comprises a built-in wireless communication module to identify the user.

Favourably the head and the front panel are arranged on the circumference of blinking LEDs configured to indicate a current state of the device, range of motion and/or other data.

The main advantage of the invention is the possibility of individual adjustment of the range (speed) and rotation angle of the arm depending on the force (character) of the signal received from the electromyograph and the program set in the remote control terminal. Precise measurement of the EMG signals makes it possible to adjust the exercises perfectly to the specific needs and abilities of the patient. At the same time, by using a remote control terminal which can be connected to more than one control motor, the work of a physiotherapist can be optimized, as it is possible to connect one remote control terminal to a few devices at the same time and s/he is then able to supervise a few devices adjusting their parameters as needed. It not only makes the work more effective but also lowers the cost of rehabilitation. The communication between the remote control terminal and the rehabilitation devices may be direct: by means of a radio or cable connection, or by means of intermediate devices mediating in the exchange of measurement and control data. In particular the intermediate units may be additionally responsible for storing, visualizing or emergency control.

The device according to the invention makes it possible to adjust the range (speed) and the rotation angle of the arm to the force (character) of the signal received from the electromyograph sensor and the program set in the remote control terminal. The openings made in the head from the side of the arm on the circumference of the column crown after insertion of pins mechanically block the range of movement of the arm.

Mounting the base on at least three wheels and the use of telescopically folded arm of the base detachable from the column ensures a wide range of rotation which makes rehabilitation of practically any joint possible. At the same time it is characteristic of the invention that there may be a force sensor built in between the motor and the mounting of the arm, which adjusts the movement of the motor to the force generated by the user's limb, which supports its movement. In particular such feedback may generate dynamic resistance, demanding from the user a specifically defined force value. The device also makes it possible to display, by means of an intermediate module, a specific picture on the screen, which depends on the movement made by the person being rehabilitated.

The hereby device, by using electromyograph to measure the level of muscular tension and software to transform the results into the movement of the device supporting the mechanical movement of the limb within the range dependent on the EMG level, enables effective muscular rehabilitation. At the same time, by using the telescopically folded arm mounted on a column with adjustable height the device may be adjusted to any patient's individual needs for the rehabilitation of both upper and lower limbs. In particular, arms mounted on the column are adjusted to a specific joint and may be exchanged depending on the rehabilitated limb.

The subject of the invention in one embodiment is presented in the drawing where:

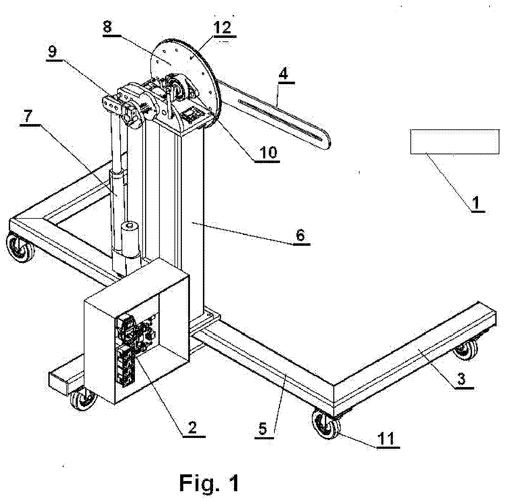

Fig. 1 presents the diagram of the whole rehabilitation device

### Embodiment 1

A rehabilitation device comprising a remote control terminal 1, a base 3, an arm 4 mounted to a column 8 with adjustable height containing a motor 7 propelling the arm 4 moving within the angular range of 360° and two electrodes of the electromyograph (EMG) sensor 10. The EMG signal from the sensor connected to the muscle or muscles of the patient is transmitted to the network of the motor controlling module and to a remote control terminal 1 regulating the adjustment of the range of movement to the muscular tension thus programming the movement of the arm on a set radius within the range of 120° while the range (speed) and angle of rotatation of the arm 4 depends on the force (character) of the signal received from the electromyograph sensor 10 and the program set in the remote control terminal 1. In the head 8 of the device, from the side of the arm 4, on the circumference of the column crown 6, there are openings 12 to enable mechanical blocking of the movement of the arm 4. Between the end of the motor 7 and the mounting of the arm 4 there is a built-in force sensor 9. The base 3 is mounted on three wheels 11 and its middle part 5 is telescopically foldable.

### Embodiment 2

The device comprises of the elements as in embodiment 1 and additionally the arm 4 is telescopically foldable. The arm 4 is detachably mounted on the column 6.

### Embodiment 3

A device as described in embodiments 1 and 2 equipped with an additional intermediate module and a monitor where a picture is displayed (e.g.: of a computer game) depending on the movements made by the patient being rehabilitated.

The embodiments presented above do not exhaust the possibilities of using the invention. The invention is defined by the appended claims.

## Claims

1. A rehabilitation device used to rehabilitate weakened muscular tension comprising a remote control terminal, a base, an extension mounted to a column with adjustable height containing an motor configured to propel the extension and at least two electrodes of the electromyography (EMG) sensor, wherein the motor (7) is configured to enable the extension (4) to move within the angular range of 360°, while the EMG signal from the sensor connected to the muscle or muscles of the patient is transmitted to the network of the motor controlling module (2), and then further by radio or cable connection to a remote control terminal (1);
wherein the controlling module (2) is configured to provide individual adjustment of the speed and rotation angle of the extension depending exclusively on the character of the signal received from the EMG and a program set in the remote control terminal.

2. A device according to claim 1, wherein the base (3) is mounted on at least three wheels (11).

3. A device according to claim 1, wherein the extension (4) is telescopically folded.

4. A device according to claim 1, wherein the extension (4) is detachably mounted on the column (6).

5. A device according to claim 1, wherein a force sensor (9) is built in between the end of the motor (7) and the extension (4) mounting.

6. A device according to claim 1, wherein an intermediate module is configured to display a picture on a screen depending on the movement made by the patient being rehabilitated.

7. A device according to claim 1, wherein the extension (4) is exchangeable and its size is adjusted to rehabilitating a specific limb.

8. A device according to claim 1, wherein the detachable extension (4) has an electronic module supplied from the device that are configured to identify extension.

9. A device according to claim 8, wherein the detachable extension (4) has a means to power a drive and the data derived from this process are passed to the network engine control module (2).

10. A device according to claim 1, wherein the extension (4) can be blocked by an electromagnet device.

11. A device according to claim 1, wherein mechanically or electrically retractable pins (12) configured to lock the range of movement of the extension (4) are arranged on the circumference of the front head (8) of the device.

12. A device according to claim 1, wherein the base (3) comprises replaceable batteries to power the device.

13. A device according to claim 12 wherein the batteries in the base (3) have integrated antennas for wireless charging.

14. A device according to claim 1, comprising a built-in wireless communication module to identify the user.

15. A device according to claim 1, wherein the head (8) of the device and the front panel (6) are arranged on the circumference of blinking LEDs configured to indicate a current state of the device, range of motion and/or other data.

## Patentansprüche

1. Ein Rehabilitationsgerät zur Rehabilitation geschwächter Muskelspannung bestehend aus einem Fernbedienungsterminal, einem Aufbau, einer an einer Säule mit einstellbarer Höhe angebrachten Erweiterung, die einen für den Antrieb der Erweiterung konfigurierten Motor und mindestens zwei Elektroden des Elektromyographie-Sensors (EMG) enthält, wo der Motor (7) konfiguriert ist, um der Erweiterung (4) Bewegung im Winkelbereich von 360° zu ermöglichen, während das EMG-Signal vom Sensor, der mit dem Muskel oder den Muskeln des Patienten verbunden ist, zum Netzwerk des Motorsteuerungsmoduls (2) und dann mit einer Funk- oder Kabelverbindung weiter zum Fernbedienungsterminal (1) übertragen wird;
Wo das Steuerungsmodul (2) konfiguriert ist, um eine individuelle Anpassung der Geschwindigkeit und des Drehwinkels der Erweiterung allein von dem Charakter des vom EMG erhaltenen Signals und der Programm im Fernbedienungsterminal abhängig zu ermöglichen.

2. Ein Gerät gemäß Anspruch 1, wo der Aufbau (3) auf mindestens drei Rädern (11) aufgesetzt ist.

3. Ein Gerät gemäß Anspruch 1, wo die Erweiterung (4) teleskopisch zusammengesetzt ist.

4. Ein Gerät gemäß Anspruch 1, wo die Erweiterung (4) ablösbar an die Säule (6) angebracht ist.

5. Ein Gerät gemäß Anspruch 1, wo der Kraftsensor (9) zwischen dem Ende des Motors (7) und die Anbringung der Erweiterung (4) eingebaut ist.

6. Ein Gerät gemäß Anspruch 1, wo ein Zwischenmodul konfiguriert ist, um ein Bild auf dem Bildschirm abhängig von der Bewegung des Patienten während der Rehabilitation auszustrahlen.

7. Ein Gerät gemäß Anspruch 1, wo die Erweiterung (4) austauschbar und ihre Größe an die Rehabilitation eines bestimmten Gliedes angepasst sind.

8. Ein Gerät gemäß Anspruch 1, wo die ablösbare Erweiterung (4) mit einem durch das Gerät bedienten elektronischen Modul ausgestattet ist, das für die Identifikation der Erweiterung konfiguriert ist.

9. Ein Gerät gemäß Anspruch 8, wo die ablösbare Erweiterung (4) mit Mitteln zur Versorgung eines Laufwerks ausgestattet ist und die aus diesem Prozess stammenden Daten zum Netzwerkantriebssteuerungsmodul (2) weitergeleitet werden.

10. Ein Gerät gemäß Anspruch 1, wo die Erweiterung (4) mit einer elektromagnetischen Vorrichtung geblockt werden kann.

11. Ein Gerät gemäß Anspruch 1, wo mechanisch oder elektrisch einschiebbare Nadeln (12) zur Einsperrung des Bewegungsbereiches der Erweiterung (4) auf dem Umkreis des Kopfstückes (8) des Gerätes eingerichtet sind.

12. Ein Gerät gemäß Anspruch 1, wo der Aufbau (3) austauschbare Akkus zum Antrieb des Gerätes enthält.

13. Ein Gerät gemäß Anspruch 12, wo die Akkus im Aufbau (3) mit eingebauten Antennen für kabelloses Aufladen ausgestattet sind.

14. Ein Gerät gemäß Anspruch 1, das mit einem eingebauten kabellosen Kommunikationsmodul zur Identifizierung der Benutzer ausgestattet ist.

15. Ein Gerät gemäß Anspruch 1, wo das Kopfstück (8) des Gerätes und das vordere Profil auf dem Umfang mit blinkenden LED ausgestattet sind, die zur Anzeige des aktuellen Status des Gerätes, des Bewegungsbereiches und/oder anderen Daten eingerichtet sind.

## Revendications

1. Dispositif de rééducation utilisé pour rééduquer une tension musculaire affaiblie comprenant un terminal de télécommande, une base, une extension montée sur une colonne à hauteur réglable contenant un moteur configuré pour propulser l'extension et au moins deux électrodes du capteur d'électromyographie (EMG), dans lequel le moteur (7) est configuré pour permettre à l'extension (4) de se déplacer dans les limites de la plage angulaire de 360°, tandis que le signal EMG provenant du capteur connecté au muscle ou aux muscles du patient est transmis au réseau du module de commande de moteur (2), puis par liaison radio ou par câble à un terminal de télécommande (1) ; dans lequel le module de commande (2) est configuré pour fournir un réglage individuel de la vitesse et de l'angle de rotation de l'extension en fonction exclusivement du caractère du signal reçu de l'EMG et d'un programme défini dans le terminal de télécommande.

2. Dispositif selon la revendication 1, dans lequel la base (3) est montée sur au moins trois roues (11).

3. Dispositif selon la revendication 1, dans lequel l'extension (4) est repliée de manière télescopique.

4. Dispositif selon la revendication 1, dans lequel l'extension (4) est montée de manière détachable sur la colonne (6).

5. Dispositif selon la revendication 1, dans lequel un capteur de force (9) est intégré entre l'extrémité du moteur (7) et le montage d'extension (4).

6. Dispositif selon la revendication 1, dans lequel un module intermédiaire est configuré pour afficher une image sur un écran en fonction du mouvement effectué par le patient en cours de rééducation.

7. Dispositif selon la revendication 1, dans lequel l'extension (4) est interchangeable et sa taille est réglée pour rééduquer un membre spécifique.

8. Dispositif selon la revendication 1, dans lequel l'extension détachable (4) a un module électronique fourni par le dispositif qui est configuré pour identifier l'extension.

9. Dispositif selon la revendication 8, dans lequel l'extension détachable (4) a un moyen d'alimenter un entraînement et les données dérivées de ce processus sont transmises au module de commande de moteur de réseau (2).

10. Dispositif selon la revendication 1, dans lequel l'extension (4) peut être bloquée par un dispositif électromagnétique.

11. Dispositif selon la revendication 1, dans lequel des ergots (12) rétractables mécaniquement ou électriquement configurés pour verrouiller la plage de déplacement de l'extension (4) sont agencés sur la circonférence de la tête avant (8) du dispositif.

12. Dispositif selon la revendication 1, dans lequel la base (3) comprend des batteries remplaçables pour alimenter le dispositif.

13. Dispositif selon la revendication 12, dans lequel les batteries dans la base (3) ont des antennes intégrées pour une charge sans fil.

14. Dispositif selon la revendication 1, comprenant un module de communication sans fil intégré pour identifier l'utilisateur.

15. Dispositif selon la revendication 1, dans lequel la tête (8) du dispositif et le panneau avant (6) sont agencés sur la circonférence de DEL clignotantes configurées pour indiquer un état actuel du dispositif, la plage de déplacement et/ou d'autres données.
